# EUROPÄISCHE PATENTSCHRIFT

(11) **EP 1 871 266 B1**
(45) Veröffentlichungstag und Bekanntmachung des Hinweises auf die Patenterteilung: **04.03.2015**
(21) Anmeldenummer: 06723529.1
(22) Anmeldetag: 17.03.2006
(51) Int. Cl.: A61B 19/00

(54) **MARKER FÜR MENSCHLICHES UND TIERISCHES GEWEBE INSBESONDERE WEICHTEILGEWEBE**
MARKER FOR HUMAN OR ANIMAL TISSUES, IN PARTICULAR FOR SOFT TISSUES
MARQUEUR POUR TISSU HUMAIN OU ANIMAL, EN PARTICULIER POUR TISSUS MOUS

(30) Priorität: 18.03.2005 DE 102005012574
(43) Veröffentlichungstag der Anmeldung: 02.01.2008
(73) Patentinhaber: BKH Technotransfer Gmbh, 82288 Kottgeisering (DE)
(72) Erfinder: HESKE, Norbert, 82299 Türkenfeld (DE)
(74) Vertreter: Rösler, Uwe
(86) Internationale Anmeldenummer: PCT/EP2006/002496
(87) Internationale Veröffentlichungsnummer: WO 2006/097331

(56) Entgegenhaltungen:
- WO-A-2006/000568
- US-A- 6 053 925
- US-A1- 2001 023 322
- US-A1- 2005 059 888

## Beschreibung

Die Erfindung betrifft einen Marker für menschliches oder tierisches Gewebe, insbesondere Weichteilgewebe, der aus vorprogrammierbarem Material (z.B. Nickel-Titan-Draht) ringförmig gefertigt wird, der sich nach einer ihm aufgezwungenen Längserstreckung nach Freigabe in die Ausgangsform, die vorprogrammierte Ringform, zurückbildet.

Weiterhin betrifft die Erfindung ein Herstellungsverfahren für den Marker.

Marker der verschiedensten Art und aus verschiedenen Materialien sind bekannt; sie werden zur Kennzeichnung von Tumoren in menschlichem oder tierischem Weichteilgewebe verwandt. Nach einer Brustbiopsie werden die Marker z. B. mittels einer Kanüle in den Biopsietubus eingeführt und nach Erreichen der gewünschten Lage mittels des Mandrins (Nadel) aus der Kanüle ausgestoßen. Das Ausstoßen erfolgt seitlich oder an der distalen Spitze der Kanüle.

Die Verwendung derartiger bekannter Marker setzt im Allgemeinen voraus, dass vor dem Einführen eine Öffnung (Tubus) gemacht wird, in die die Kanüle mit dem Marker eingeführt werden kann.

Es ist auch bekannt, den Marker unmittelbar mittels einer Art Spritzennadel in die Weichteile einzusetzen, was ebenfalls durch Ausstoßen des Markers aus der Hohlnadel mittels eines darin geführten Mandrins erfolgt. Es ist auch die Verwendung von vorprogrammierbarem Drahtmaterial für Marker bekannt..

Neuerdings ist die medikamentöse Behandlung von Tumoren weiterentwickelt worden, insbesondere ist es gelungen, Tumore medikamentös zur verkleinern (downstaging). Unter Umständen sind die Tumore nach der Behandlung nicht mehr sichtbar. Auch hier werden zur Beobachtung des Behandlungserfolges Marker eingesetzt.

All diese Marker weisen den Nachteil auf, dass sie bei Röntgen-, Computertomograph- oder Magnetresonanzverfahren und bei Ultraschallaufnahmen gar nicht, oder sehr schwer erkennbar sind. Auch haben sie gerade bei Magnetresonanzverfahren den Nachteil, dass sie im Bereich des Markers die Magnetlinien negativ beeinflussen und Bildteile löschen, bzw. durch Störeinflüsse die Gewebeausbildung in diesen Bereichen nicht erkennbar ist.

Der US 6,053,925 ist ein Gewebemarker für menschliches Gewebe zu entnehmen, das zwei miteinander verdrillten Drähten aus Formgedächtnismetall besteht. Der Markerbereich, der in einem zu markierenden Gewebebereich möglichst ortsunveränderlich zu platzieren ist, weist eine ring- oder spulenförmige Formgebung auf. Dieses Dokument offenbart die im einführenden Teil des Anspruchs 1 definierten Merkmale.

Der US 2005/0059888 A1 ist ein Marker zu entnehmen, der den Ort eines intrakorporal verbrachten biologischen Absorberkörper markiert. Der Marker besteht aus einem im Wege der Mammografie, Radiologie und Ultraschalluntersuchung detektierbaren Material bspw. aus einem Draht, der an dem Absorberkörper angebracht ist.

Der US 2001/0023322 A1 ist eine kanülenartige Positioniereinheit für einen intrakorporal einbringbaren Marker zu entnehmen. Der Marker besteht aus einem Formgedächtnismetalldraht, der sich nach intrakorporaler Positionierung zumindest an der Drahtspitze ring- oder spulenartig zu Zwecken einer festen Positionierung in einem zu markierenden Gewebebereich verformt.

Die zu lösende Aufgabe besteht darin, einen Marker herzustellen, der in menschlichem oder tierischem Gewebe, bzw. Weichteilgewebe mittels Ultraschall einerseits leicht ortbar und abbildbar ist, und der gerade bei Magnetresonanzverfahren keine negativen Auswirkungen auf den Verlauf der Magnetlinien hat, d.h. die dem Marker benachbarten Gewebeteile in ihrer Struktur erkennen lassen.

Auch soll der Bediener nach dem Einstich die Positionierung des Markers von außen zuverlässig bestimmen können. Letztlich ist für die Herstellung des Markers aus vorprogrammierbarem mehrteiligem Drahtmaterial (z.B. Nickel-Titan-Drähten) ein Verfahren zu wählen, das sicherstellt, dass die gewünschte Ringform auch nach mehreren Verformungen des vorprogrammierten mehrteiligen Drahtmaterials beim Einsetzen des Markers in das Gewebe, bzw. Weichteilgewebe sicherstellt, dass der Marker in die Ringform zurückkehrt.

Die Lösung der Aufgabe besteht darin, dass das Drahtmaterial für den Marker aus einem Mitteldraht und zwei bis sieben dünnen, um den Mitteldraht gewundenen, vorprogrammierbaren Einzeldrähten, (z.B. aus Nickel-Titan-Drähten) gebildet wird.

Durch das Umwickeln des Mitteldrahtes mit zwei bis sieben Einzeldrähten entsteht ein Drahtmaterial, das als Ausgangsmaterial für die nach einem speziellen Verfahren hergestellten ringförmigen Marker dient. Gerade dieses Drahtmaterial hat einerseits das Schallecho bei Ultraschall positiv beeinflusst und andererseits beim Magnetresonanzverfahren die negative Beeinflussung der Magnetlinien nahezu vollständig beseitigt. Ein ganz besonders gutes Ergebnis wird erzielt, wenn um den Mitteldraht z. B. drei dünne Einzeldrähte so gewunden werden, dass eine gleichmäßige durchgehende Oberfläche entsteht.

Es hat sich gezeigt, dass die einzelnen verwendeten, dünnen Drähte einen Querschnitt von 0,1 mm bis 0,5 mm aufweisen sollten, um eine hervorragende Abbildung zu erzielen. Die relativ kleinen Ringmarker mit einem Durchmesser von nur ca. 2,5 - 4,0 mm kehren bei einem derartigen elastischen Drahtmaterial leicht in ihre vorprogrammierte Ringform zurück. Ist die Drahtstärke zu groß, so wird der Rückstelleffekt evtl. negativ beeinflusst.

Sehr gut eignet sich Ausgangsdrahtmaterial mit kreisrundem Querschnitt, das wegen seines geringen Querschnitts als Vollmaterial eingesetzt wird.

Von Vorteil beim Einfädeln, aber auch für die Formstabilität ist, dass an einem Ende die einzelnen Drähte z.B. mittels Schweißen, verbunden sind, so dass das Material sich zu einer Seite hin frei bewegen kann.

Durch die Ausbildung der Kanüle der Einbringvorrichtung als Stechkanüle im Sinne einer Spritzenkanüle, kann der Arzt unmittelbar in die Weichteile einstechen, also ohne vorherige Tubierung der Weichteile. Die Kanülenspitze der Einbringvorrichtung kann z.B. über Ultraschall oder mittels Magnetresonanz genau an die zu kennzeichnende Stelle gesetzt werden, sofern sie aus dem allgemein üblichen medizinischen Metallwerkstoff besteht, um danach den vorprogrammierten Marker mittels des Mandrins an der distalen unteren Spitze der Kanüle auszustoßen, so dass er sich in seine vorgegebene Ebene eindreht..

Die Einzeldrähte, (Mitteldraht und um diesen gewundene weitere Einzeldrähte) des Markers bestehen aus einer Nickel-Titan-Legierung, einer Legierung mit einem Formgedächtnis, welche z. B. die Firma Memory-Metalle GmbH in Weil am Rhein als Draht mit kreisförmigem Querschnitt und als Vollmaterial vertreibt. Das Material hat ein Formgedächtnis. Dies bedeutet, dass der nach einem speziellen Verfahren zu einem Kreis geformte Marker, der im Kanüleninnenraum als längsgestrecktes Drahtstück zur Einführung in die Weichteile abgelegt ist, nach dem Ausstoßen aus der Kanüle das Bestreben hat, in die Ringform zurückzukehren. Beim Einfügen des Ringmarkers in das Kanülenrohr ist darauf zu achten, dass der Marker so eingefügt wird, dass der Ringmarker bei seinem Austritt am unteren Rand der Kanülenspitze den Ring zu bilden beginnt, was die spezielle Ausbildung der Kanülenspitze begünstigt. Es ist weiterhin darauf zu achten, dass das verschweißte Ende zuerst in die Kanüle eingeführt wird.

Nach dem Ausstoßen nimmt der aus Einzeldrähten bestehende Marker seine vorprogrammierte Kreisform ein und tritt in die vorbestimmte Richtung (Ebene) aus. Auf diese Weise kann die Lage von Tumoren in präziser Weise gekennzeichnet werden.

Um aus den aus zwei bis sieben dünnen Einzeldrähten, die um einen Mitteldraht zu einem Draht gewunden werden, einen ringförmigen Marker zu fertigen, ist ein spezielles Herstellungsverfahren erforderlich.

Dabei ist es wichtig, dass nach einem ersten Schritt, nämlich dem Umwickeln des Mitteldrahtes mit zwei bis sieben dünnen Drähten unter einem vorgegebenen Steigungswinkel ein Drahtstück erzeugt wird, das, um ein Aufdrehen zu verhindern, fixiert wird. Anschließend wird in einem weiteren Schritt dieses Drahtstück oder die erzeugte Litze z. B. über einen Dom mit dem gewünschten Durchmesser gewickelt und anschließend fixiert, so das kein Aufdrehen mehr möglich ist. Durch die in einem weiteren Schritt durchgeführte Wärmebehandlung wird gewissermaßen das Gedächtnis des Ausgangsmaterials (das zu einer Spirale aufgewickelte Drahtstück) soweit gelöscht, dass es kein Bestreben zeigt, dass z.B. die Einzeldrähte in ihre gestreckte Ausgangslage zurückkehren, bzw. die Spirale sich aufdrillt. Die so erzeugte Spirale kann nun in einzelne Ringmarker zerschnitten werden. Dies geschieht vorteilhafterweise z.B. mittels Laserstrahl; um das Laserstrahlverfahren durchführen zu können, wird in die Spirale z.B. ein Dorn eingebracht, der so gestaltet ist, dass beim Trennvorgang das Markermaterial mit dem Dom einerseits nicht verschweißt wird, andererseits bei jedem Trennvorgang am einen Ende die Einzeldrähte des Drahtmaterials verschweißt werden, während das andere Ende offen bleibt. Dadurch ist es möglich, dass bei der Aufbiegung des Markers in die gestreckte Lage, die Einzeldrähte an einem Ende zusammengehalten werden und am anderen, dem nicht verschweißten Ende, sich frei ausdehnen können. Beim Einlegen des einzelnen Ringmarkers in die distale Spritzenkanüle, was durch Aufbiegen des Rings geschieht, und wobei aus dem Ringmarker ein längsgestrecktes mehrteiliges Drahtstück entsteht, ist darauf zu achten, dass der Marker beim Ausstoßen aus der Kanüle am unteren distalen Spitzenpunkt beginnt in seine Ringform zurückzukehren.

Beim Einlegen des ringförmigen Markers in die Spritzenkanüle, von der Spitze her, ist daher darauf zu achten, dass die Austrittsebene des Ringmarkers senkrecht zur schräg abgeschnittenen Ebene der Kanüle steht, so dass sich der Ringmarker in das Gewebe einrollen kann. Der Ringmarker wird also gezielt in eine Ebene eingelegt, die senkrecht zur Ebene der abgeschnittenen Ebene der Kanülenspitze steht. Eventuelle Abweichungen von dieser Ebene werden durch die schräggeschnittene Spitze korrigiert; auch verhindert die spezielle Spitzenausbildung, dass der Marker verkehrt zur Austrittsebene eingelegt wird.

Nachfolgend sind Ausführungsbeispiele des erfindungsgemäßen Markers, der Einbringvorrichtung sowie eines Herstellungsverfahrens zum Teil anhand von Zeichnungen näher erläutert. Es zeigt:

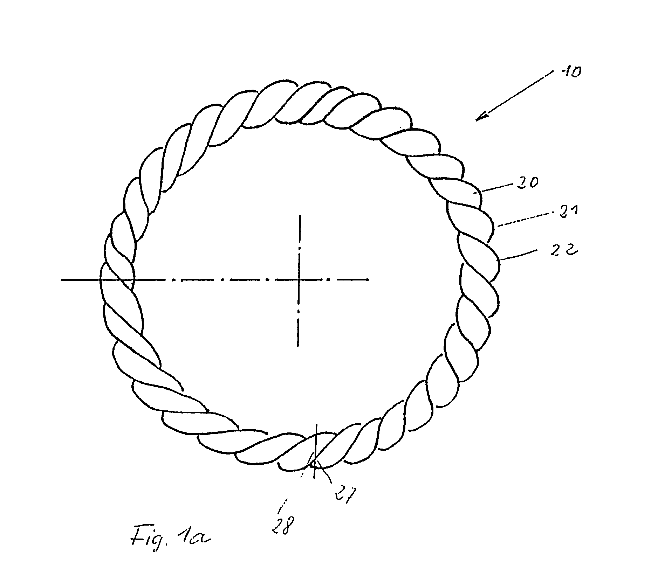

- Fig. 1a: einen Ringmarker aus drei um einen Mitteldraht gewundenen Drähten in geschlossener Form

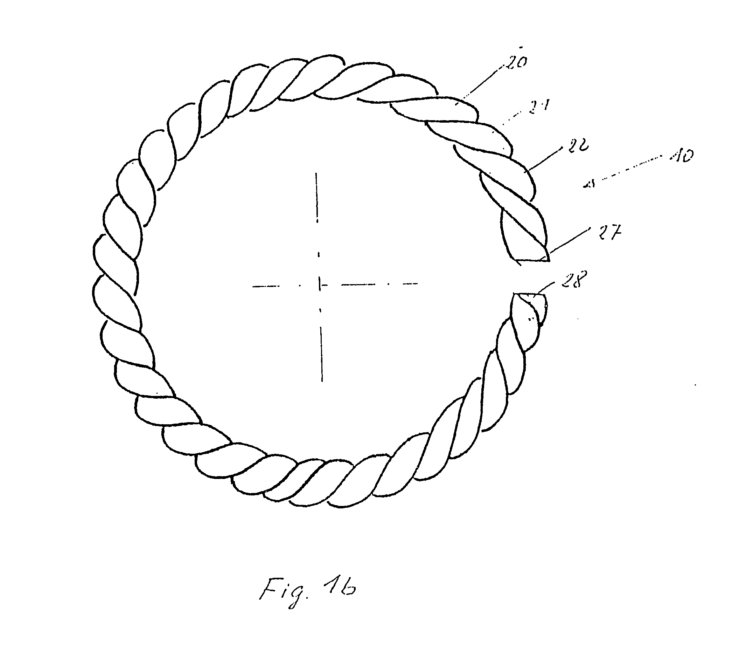

- Fig. 1b: einen Ringmarker mit dem Drahtmaterial wie Fig. 1a, jedoch in offener Bauweise

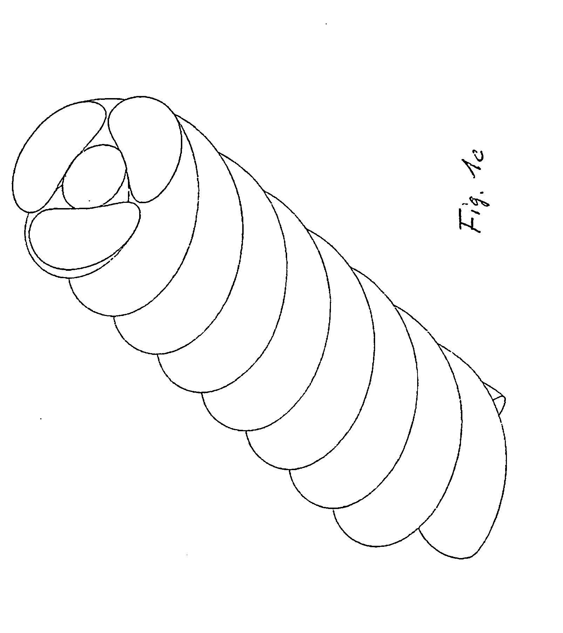

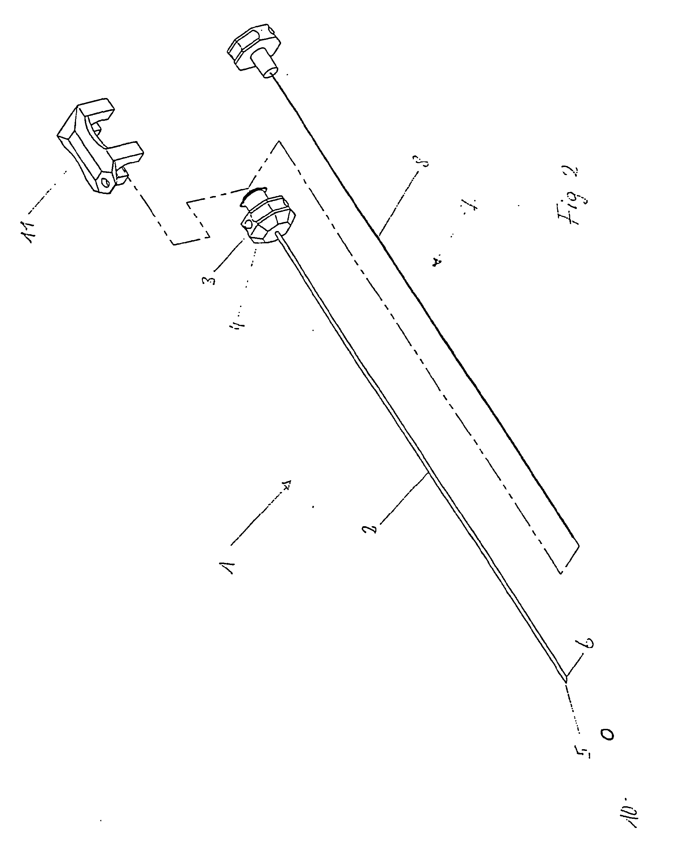

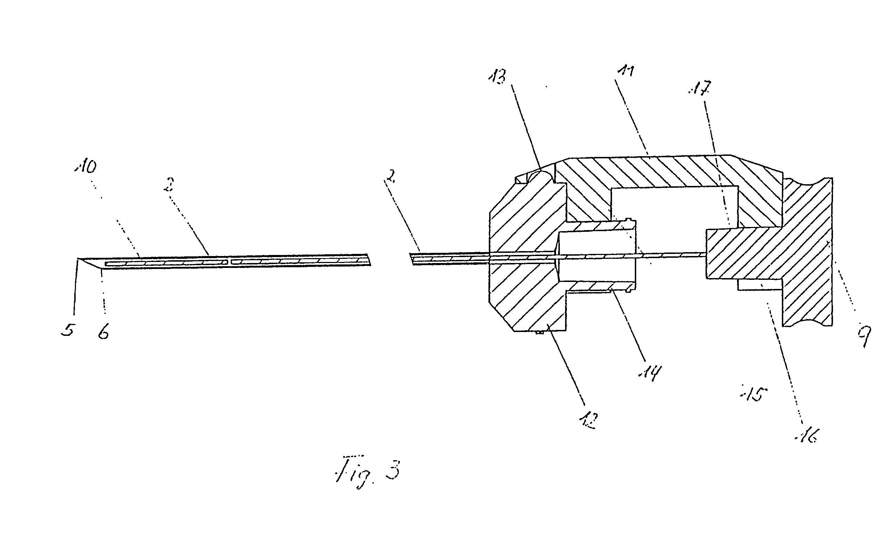

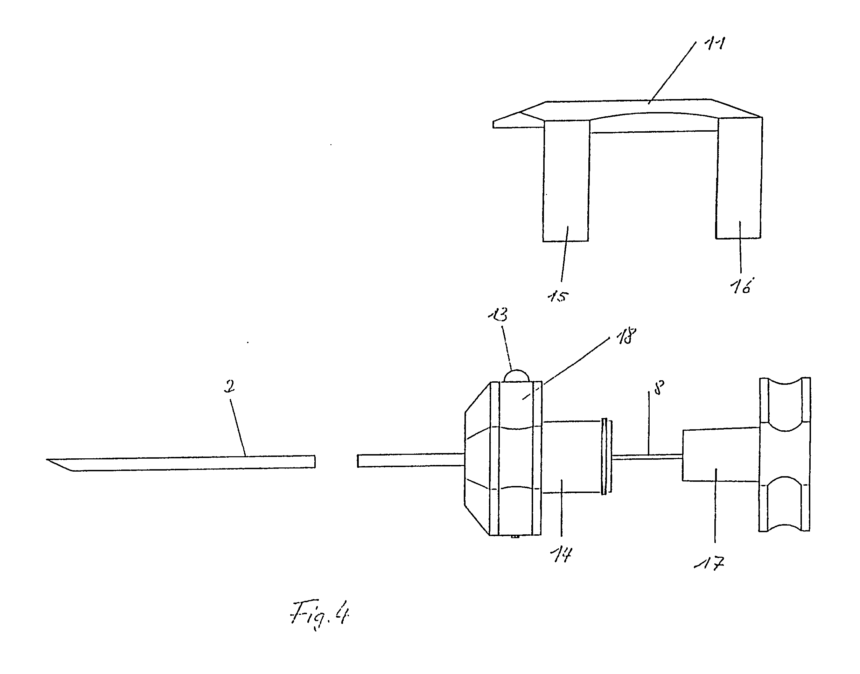

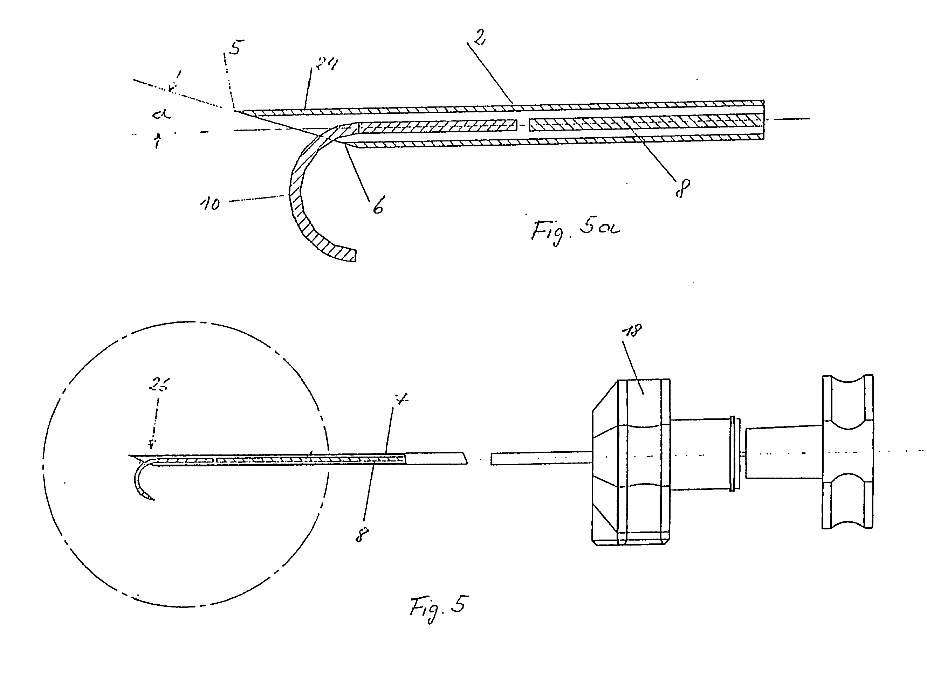

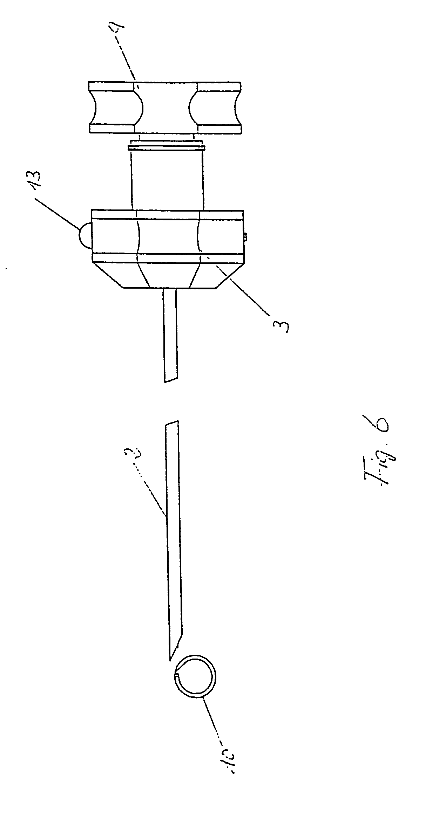

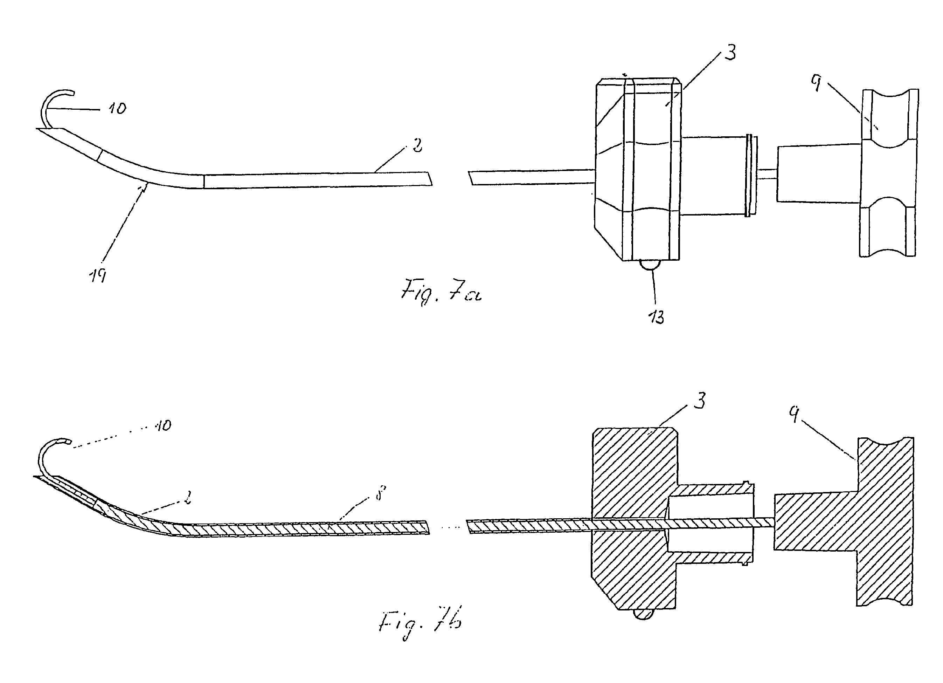

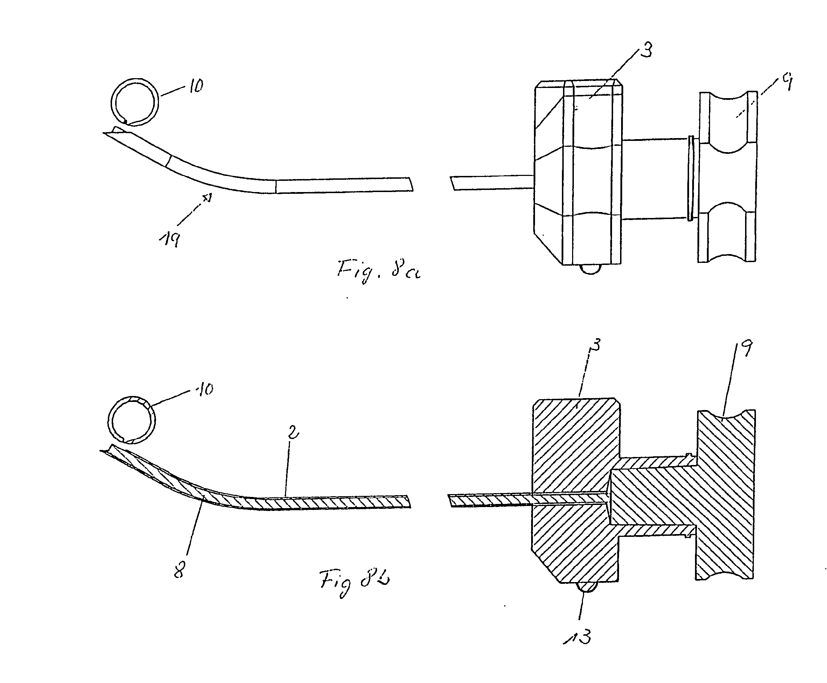

- Fig. 1c: Ausschnitt aus dem Drahtmaterial mit einem Mitteldraht und drei darum gewundenen Drähten und
- Fig. 1d: Ausschnitt aus dem Drahtmaterial.

Figur 1a zeigt einen geschlossenen Ringmarker, d.h. die beiden Endstücke 27, 28 stoßen stumpf aufeinander. Wie Fig. 1b zeigt, können die beiden Endstücke 27, 28 auch einen geringen Abstand zueinander haben. Sie können sich auch geringfügig überlagern oder sind seitlich zueinander versetzt.

Der Ringmarker hat im Allgemeinen einen Innendurchmesser von ca. 2,0 - 2,5 mm und einen Außendurchmesser von 2,5 - 5 mm, je nach Stärke der verwendeten Einzeldrähte und dem gewünschten Durchmesser des Ringmarkers. Das aus einem Mitteldraht und zwei bis sieben um diesen Mitteldraht gleichmäßig gewundenen, dünnen Einzeldrähten erzeugte Drahtmaterial für die verschieden gestalteten Ringmarker 10 hat einen Drahtdurchmesser von 0,1 - 0,5 mm. Die Einzeldrähte des Drahtmaterials (Vollmaterial) können gleichen Querschnitt haben, wie insbes. Fig. 1c zeigt. Um einen im Querschnitt kreisförmigen Mitteldraht 29 sind im gezeigten Fall drei ebenfalls kreisförmige Einzeldrähte 20, 21, 22 spiralförmig gleichmäßig, mit gleichem Steigungswinkel gewunden, so dass eine gleichmäßige Außenoberfläche (sh. Fig., 1 c) entsteht. Im gezeigten Beispiel sind alle Drahtquerschnitte gleich, bei allen ist Vollmaterial verwendet. Das verwendete Material ist eine Titan-NickelLegierung mit Formgedächtnis, wie es am Markt angeboten wird.

Statt einem kreisförmigen Querschnitt können die verwendeten Einzeldrähte auch andere Querschnitte, z.B. ovale oder viereckige usw. aufweisen. Es kann auch Hohlmaterial zum Einsatz kommen. Wichtig ist, dass das Schallecho bei Verwendung von Ultraschall den Marker deutlich im Bild erscheinen lässt. Gleiches gilt für das MR-Verfahren; die Magnetlinien dürfen durch den eingelegten Marker nicht die angrenzenden Bereiche auslöschen. Wichtig ist auch, dass das Ausgangsmaterial eine gewisse Elastizität aufweist um Ringmarker formen zu können, und die erzeugten Ringmarker in die Kanüle der Einbringvorrichtung einfädeln zu können.

Die Auslegung des Ringmarkers insgesamt muss so erfolgen, dass er sowohl den Einsatz von Ultraschall- wie den Einsatz von MR-Verfahren ohne schädigende Wirkung der Bildqualität zulässt. Dies hängt vor allem von der Auswahl des Grundmaterials, dem Drahtmaterial, ab. Wie bereits beschrieben, besteht das Drahtmaterial für den Ringmarker aus einem Mitteldraht 29 und z. B. drei um diesen gewundenen Einzeldrähten 20,21,22. Die Steigungswinkel der Einzeldrähte 20, 21, 22 sind hierbei so zu wählen, dass ein Drahtmaterial entsteht, bei dem die Einzeldrähte eng aneinander liegen, wie es bei Seilen üblich ist, und der erzeugte Draht eine gleichmäßige Oberfläche aufweist. Bezüglich der Enden 27, 28 der von den Drahtstücken erzeugten Ringmarker (wie später im Einzelnen beschrieben) wäre zu erwähnen, dass an einem Ende des von einer Spirale abgetrennten einzelnen Ringmarkers die einzelnen Drähte miteinander verschweißt sind, so dass nur an einem Ende die Drähte frei enden und bei einer Längserstreckung sich zu diesem Ende hin ausdehnen können..

Um einen ringförmigen Marker aus Nickel-Titan-Material mit Formgedächtnis herzustellen bedarf es eines speziellen Herstellungsverfahrens. In einem ersten Schritt werden die äußeren Drähte 20, 21, 22 (zwei bis sieben) um einen Mitteldraht 29 gewunden und so ein gleichmäßig umwundenes, Drahtseil mit konstantem Durchmesser erzeugt. Nach der Herstellung des Drahtseils wird dieses so fixiert, dass die drei einzelnen äußeren Drähte sich, insbesondere wegen des Rückstelleffekts aufgrund des Formgedächtnisses, nicht aufdrehen können. Das fixierte Drahtseilstück wird anschließend z. B. auf einen Dorn aufgewickelt um eine Spirale zu erzeugen. Auch diese aus dem Drahtseil geformte Spirale wird fixiert um ein Aufdrehen zu verhindern. Der Domdurchmesser entspricht in etwa dem Innendurchmesser des gewünschten späteren, ringförmigen Markers. Das auf den Dom aufgewickelte Drahtseilstück, die Spirale, wird in seiner Lage fixiert und wird nun einer Wärmebehandlung unterzogen. Das Drahtseilstück wird hierbei auf eine Temperatur gebracht, die so hoch ist, dass das Titan-Nickel-Material sein Formgedächtnis verliert, und hat somit nach dieser Wärmebehandlung nicht mehr das Bestreben in seine Ausgangsform, in einen geraden Draht ,zurückzukehren. Nachdem das Drahtseilstück abgekühlt ist, können die ringförmigen Marker von der Drahtsseilspirale abgetrennt werden.

Zum Abtrennen, bzw. Abschneiden der Ringmarker von der Spirale z. B. mittels Laserstrahl, wird die Spirale auf einen Dom aufgezogen, der so gestaltet sein muss, dass einerseits der Laserstrahl den Marker von der Spirale abtrennt (abschneidet) jedoch nicht gleichzeitig mit dem Dom verschweißt. Andererseits soll der Laserstrahl bei der Durchtrennung auf einer Seite der Schnittstelle die Drähte verschweißen, während diese auf der gegenüber liegenden Schnittstelle frei bleiben. Dies hat den Vorteil, dass die Einzeldrähte an einem Ende 27 des Markers fest miteinander verbunden sind, sich jedoch bei dem Einfädelungsprozess in die Kanüle frei zum nicht verschweißten Ende 28 bewegen können. Natürlich kann der Verschweißungsprozess auch getrennt vom Abschneidprozess durchgeführt werden. Wichtig ist allein, dass an einem Ende die Drahtenden miteinander verbunden sind.

Aufgrund seiner Form und seines Aufbaus wandert der Ringmarker nicht. Er ist wegen der verdrillten Drähte, die eine gute Schallresonanz geben, im Ultraschallbild leicht zu erkennen. Beim Magnetresonanzverfahren ist die negative Beeinflussung der Magnetlinien nahezu eliminiert. Das Gesagte gilt auch für andere Verfahren, wie Röntgen und Computertomographie.

### Bezugszeichenliste

- 10: Marker (Ringmarker)
- 20: Einzeldraht
- 21: Einzeldraht
- 22: Einzeldraht
- 27: Endstücke
- 28: Endstücke
- 29: Mitteldraht

## Patentansprüche

1. Marker für tierisches oder menschliches Gewebe, insbesondere Weichteilgewebe, der aus vorprogrammierbarem Material (z.B. Nickel-Titan-Draht) ringförmig gefertigt wird, der sich nach einer ihm aufgezwungenen Längserstreckung nach Freigabe in die Ausgangsform, die vorprogrammierte Ringform zurückbildet, **dadurch gekennzeichnet, dass** das Drahtmaterial für den Marker (10) aus einem Mitteldraht und zwei bis sieben weiteren, dünnen um den Mitteldraht gewundenen, vorprogrammierbaren Einzeldrähten (20, 21, bzw. 22), (z.B. Nickel-Titan-Drähten) gebildet wird.

2. Marker nach Anspruch 1, **dadurch gekennzeichnet, dass** drei Einzeldrähte (20, 21, bzw. 22) um den Mitteldraht gewunden werden

3. Marker nach Anspruch 1 und 2, **dadurch gekennzeichnet, dass** bei der Herstellung des Drahtmaterials für den Marker (10) die Einzeldrähte einen Querschnitt von 0,1 mm bis 0,5 mm haben.

4. Marker nach einem oder mehreren der vorhergehenden Ansprüche, **dadurch gekennzeichnet, dass** die vorprogrammierte Form eine Ringform ist, so dass der in das Weichteilgewebe gesetzte Marker (10) aus elastischem Drahtmaterial ringförmig mit offenen oder überlappten Enden ausgebildet ist.

5. Marker nach einem oder mehreren der Ansprüche 1 bis 5, **dadurch gekennzeichnet, dass** der Querschnitt der verwendeten Einzeldrähte (20, 21, bzw. 22) eine Kreisform haben und aus Vollmaterial sind.

6. Marker nach einem oder mehreren der vorhergehenden Ansprüche, **dadurch gekennzeichnet, dass** die um den Mitteldraht gewundene Drähte einen an den verwendeten Draht angepassten, gleichen Steigungswinkel aufweisen, so dass eine einheitliche Oberfläche entsteht.

7. Marker nach einem oder mehreren der vorhergehenden Ansprüche, **dadurch gekennzeichnet, dass** an einem Ende die Einzeldrähte, z.B. mittels Schweißen miteinander verbunden sind.

8. Verfahren zur Herstellung des Markers nach einem oder mehreren der Ansprüche 1 - 7, **dadurch gekennzeichnet, dass** in einem ersten Schritt zwei bis sieben Einzeldrähte um einen Mitteldraht unter einem vorgegebenen Steigungswinkel gewunden werden, dass in einem weiteren Schritt das so entstandene Drahtmaterial zu einer Spirale geformt wird, so dass für die vorprogrammierte Ringform zunächst eine Spirale mit dem gewünschten Innendurchmesser entsteht, diese aus Drahtmaterial geformte Spirale fixiert wird, dass in einem weiteren Schritt das Drahtmaterial als Spirale im fixierten Zustand bei einer Temperatur wärmebehandelt wird, die das Formgedächtnis auslöscht, dass nach der Wärmebehandlung die aus Drahtmaterial erzeugte Spirale in einzelne Ringmarker zertrennt wird und gleichzeitig oder danach die Einzeldrähte des Drahtmaterials miteinander verschweißt werden.

9. Verfahren zur Herstellung des Markers nach Anspruch 8, **dadurch gekennzeichnet, dass** der Marker (10) in den distalen Teil der Kanülenspitze (19) so eingelegt wird, dass sich der Marker beim Ausstoßen aus seiner Längserstreckung auf die Seite zum unteren distalen Spitzenpunkt (6) dreht.

10. Verfahren zur Herstellung des Markers nach Anspruch 8, **dadurch gekennzeichnet, dass** die Wärmebehandlungstemperatur, je nach Wahl des Ausgangsmaterials, im Bereich von 400 ° C - 600 ° C. liegt.

## Claims

1. A marker for animal or human tissue, in particular for soft tissue which is prepared in the form of a ring from preprogammable material (e.g., nickel-titanium wire), regressing after having been released after a longitudinal elongation has been imposed on it, back to the initial shape, the preprogrammed ring shape, **characterized in that**
the wire material for the marker (10) is formed from a central wire and two to seven additional thin preprogrammable individual wires (20, 21 and/or 22) (e.g., nickel-titanium wires) coiled around the central wire.

2. The marker according to Claim 1,
**characterized in that**
three individual wires (20, 21 and/or 22) are coiled around the central wire.

3. The marker according to Claim 1 and 2,
**characterized in that**
the individual wires have a cross section of 0.1 mm to 0.5 mm in the production of the wire material for the marker (10).

4. The marker according to one or more of the preceding claims,
**characterized in that**
the preprogrammed shape is a ring shape, so that the marker (10), which is placed in the soft tissue, is formed from an elastic wire material in the shape of a ring having open or overlapped ends.

5. The marker according to one or more of Claims 1 to 5, **characterized in that**
the cross section of the individual wires (20, 21 and/or 22) that are used has a circular shape and is made of solid material.

6. The marker according to one or more of the preceding claims,
**characterized in that**
the wires coiled around the central wire have the same angle of gradient, which is adapted to the wire used, so that the result is a uniform surface area.

7. The marker according to one or more of the preceding claims,
**characterized in that**
the individual wires are joined to one another, e.g., by welding at one end.

8. A method for producing the marker according to any one or more of Claims 1 to 7,
**characterized in that**
two to seven individual wires are coiled around a central wire at a preselected angle of gradient in a first step; in an additional step, the resulting wire material is shaped into a spiral, so that for the preprogrammed ring shape, a spiral having the desired inside diameter is formed first; this spiral shaped from wire material is secured; in another step, the wire material as a spiral in the fixed condition is heat-treated at a temperature which destroys the shape memory; after the heat treatment, the spiral produced from the wire material is divided into individual ring markers, and the individual wires of the wire material are welded to one another at the same time or thereafter.

9. The method for producing the marker according to Claim 8,
**characterized in that**
the marker (10) is inserted into the distal part of the cannula tip (19), so that the marker is rotated onto the side facing the lower distal peak point (6) when ejected out of its longitudinal extent.

10. The method for producing the marker according to Claim 8,
**characterized in that**
the heat treatment temperature is in the range of 400°C-600°C, depending on the choice of the starting material.

## Revendications

1. Marqueur pour tissu animal ou humain, en particulier tissu des parties molles, qui est fabriqué en forme annulaire à partir de matériau préprogrammable (par exemple du fil de nickel-titane) et qui, après un étirement longitudinal qui lui est imposé, reprend après déblocage sa forme initiale, la forme annulaire préprogrammée, **caractérisé en ce que** le matériau en forme de fil pour le marqueur (10) est constitué d'un fil central et de deux à sept autres fils individuels préprogrammés fins enroulés autour du fil central (20, 21 ou 22) (par exemple des fils de nickel-titane).

2. Marqueur selon la revendication 1, **caractérisé en ce que** trois fils individuels (20, 21 ou 22) sont enroulés autour du fil central.

3. Marqueur selon les revendications 1 et 2, **caractérisé en ce que**, lors de la fabrication du matériau en forme de fil pour le marqueur (10), les fils individuels ont une section transversale de 0,1 mm à 0,5 mm.

4. Marqueur selon une ou plusieurs des revendications précédentes, **caractérisé en ce que** la forme préprogrammée est une forme annulaire, de sorte que le marqueur (10) posé dans le tissu des parties molles est constitué de matériau en forme de fil élastique de forme annulaire comportant des extrémités ouvertes ou se chevauchant.

5. Marqueur selon une ou plusieurs des revendications 1 à 5, **caractérisé en ce que** la section transversale des fils individuels utilisés (20, 21 ou 22) est de forme circulaire et composée de matériau plein.

6. Marqueur selon une ou plusieurs des revendications précédentes, **caractérisé en ce que** les fils enroulés autour du fil central présentent un angle de pente identique adapté au fil utilisé, de sorte qu'une surface uniforme est créée.

7. Marqueur selon une ou plusieurs des revendications précédentes, **caractérisé en ce que**, à une extrémité, les fils individuels sont reliés ensemble par exemple par soudage.

8. Procédé de fabrication du marqueur selon une ou plusieurs des revendications 1 à 7, **caractérisé en ce que**, dans une première étape, deux à sept fils individuels sont enroulés autour d'un fil central suivant un angle de pente prédéfini, que, dans une autre étape, le matériau en forme de fil ainsi obtenu est façonné en spirale, de sorte qu'on obtient d'abord pour la forme annulaire préprogrammée une spirale ayant le diamètre intérieur souhaité, que cette spirale façonnée à partir de matériau en forme de fil est fixée, que, dans une autre étape, le matériau en forme de fil subit en tant que spirale à l'état fixé un traitement thermique à une température qui efface la mémoire de forme, que, après le traitement thermique, la spirale fabriquée à partir de matériau en forme de fil est tronçonnée en marqueurs annulaires distincts et que, simultanément ou ensuite, les fils individuels du matériau en forme de fil sont soudés ensemble.

9. Procédé de fabrication du marqueur selon la revendication 8, **caractérisé en ce que** le marqueur (10) est inséré dans la partie distale de la pointe de canule (19) de manière à ce que le marqueur se tourne vers le côté en direction du point distal inférieur de la pointe (6) en cas d'éjection depuis son extension longitudinale.

10. Procédé de fabrication du marqueur selon la revendication 8, **caractérisé en ce que** la température de traitement thermique se situe, en fonction du choix du matériau initial, dans la plage de 400 °C à 600 °C.
